**Europäisches Patentamt**

**European Patent Office**

**Office européen des brevets**

(11) Veröffentlichungsnummer: **0 163 924 B1**

# (12) EUROPÄISCHE PATENTSCHRIFT

(45) Veröffentlichungstag der Patentschrift: **18.12.91**

(51) Int. Cl.5: **A61K 31/355, A61K 9/06**

(21) Anmeldenummer: **85105171.4**

(22) Anmeldetag: **27.04.85**

Die Akte enthält technische Angaben, die nach dem Eingang der Anmeldung eingereicht wurden und die nicht in dieser Patentschrift enthalten sind.

(54) **Vitaminhaltiges Mittel zur Behandlung und zum Schutz der Schleimhäute sowie Verwendung von Vitamin E zur Herstellung des Mittels.**

(30) Priorität: **02.05.84 DE 3416209**

(43) Veröffentlichungstag der Anmeldung:
**11.12.85 Patentblatt 85/50**

(45) Bekanntmachung des Hinweises auf die Patenterteilung:
**18.12.91 Patentblatt 91/51**

(84) Benannte Vertragsstaaten:
**AT BE CH DE FR GB IT LI LU NL SE**

(56) Entgegenhaltungen:
EP-A- 0 151 987   EP-A- 0 151 989
EP-A- 0 152 106   EP-A- 0 158 090
WO-A-84/01899   FR-A- 2 420 973
GB-A- 1 453 239

(73) Patentinhaber: **Ismail, Roshdy, Dr.**
**Siebengebirgs-Apotheke Siebengebirgsallee 2**
**W-5000 Köln 41 (Klettenberg)(DE)**

(72) Erfinder: **Ismail, Roshdy, Dr.**
**Siebengebirgs-Apotheke Siebengebirgsallee 2**
**W-5000 Köln 41 (Klettenberg)(DE)**

(74) Vertreter: **Werner, Hans-Karsten, Dr. et al**
**Deichmannhaus am Hauptbahnhof**
**W-5000 Köln 1(DE)**

## Beschreibung

Gegenstand der vorliegenden Erfindung ist die Verwendung von Vitamin E in Kombination mit einem oder mehreren durchblutungsfördernden Mitteln und/oder Vitamin A als Arzneimittel.

Vitamin E ist bekannt als Antioxidans und Schutzvitamin für Phosphorlipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988-1993. Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei oraler Verabreichung von 200 bis 800 mg Vitamin E an Patienten für einen Zeitraum von 1 bis 4 Tagen, deren Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. clin. 33, S. 968-971 (1980); Natt CL. Am. J. clin. nutr. 32, S. 1359-1362 (1979); Gawlik G.M. Fed. Proc. 35 (3), S. 252 (1976) und Gorash L. Bieri J.G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurden, wobei eine Normalisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan.

In den deutschen Patentanmeldungen P 34 20 738, P 34 05 928, P 34 05 239, P 34 07 025, P 34 08 260, P 34 16 162, P 34 32 881, P 34 05 240, P 34 02 930, P 34 07 024, P 34 07 026, P 34 15 250, P 34 27 193 wird ferner der Einsatz von Vitamin E zur Behandlung der Venen, des Analbereichs und von Rheumaerkrankungen vorgeschlagen.

Es ist weiterhin bekannt, daß Cholesterin in menschlicher und tierischer Haut durch Ultraviolett-Licht zu Cholesterin-alpha-oxyd, einen als Krebserreger bekannten Stoff, umgewandelt wird. Versuche mit Mäusen haben gezeigt, daß bei Verabreichung von Vitamin E und C sowie zwei weiteren Antioxidantien sich kein Cholesterin-alpha-oxyd bildet (Pharm. Indu. 36, Nr. 3 (1974) Anschel, USA).

Gegenstand der deutschen Patentanmeldung P 35 07 791.3 sind Vitamin-E sowie Kombinationen von Vitamin E mit anderen Wirkstoffen, die sich als Mittel, insbesondere zur Behandlung von Ekzemen, Hautflechte, Hautentzündungen, Juckreiz, Allergien, Faltenbildungen, Pigmentierungen der Haut und Haarausfall sowie Wunden, eignen. Darüberhinaus können diese Mittel als Schutz gegen ultraviolettes Licht und zur Förderung des Haarwuchses eingesetzt werden. Diese Mittel sind ferner als Hautschutzmittel bei Bestrahlungen, z.B. von Krebspatienten, geeignet.

Aus FR-A-2 420 973 ist ein Vitamin-E-haltiges Mittel bekannt, daß bei Erkrankungen der peripheren Gefäße, des Gehirns, des Hals-, Nasen-, Ohren-Bereichs und der Augen angewendet wird. Dieses Mittel wird jedoch nur oral oder parenteral in Form von Tabletten, Kapseln und Injektionslösungen verabreicht. Weiterhin wird es in Form von Zäpfchen verabreicht.

Aus W 083/01898 ist eine pharmazeutische Wirkstoffkombination bekannt, die Vitamin A, Vitamin E, Mandelöl, Sesamöl und Olivenöl enthält. Der Nachteil einer derartigen Zusammensetzung liegt darin, daß Vitamin E schlecht von der Haut aufgenommen wird. Außerdem hat Vitamin E mit Sicherheit keine Wirkung, da es lediglich in niedrigen Konzentrationen zugesetzt wird.

Vitamin E kann außerdem die Zellmembran vor Oxidationen schützen und die Permeabilität sowie Stabilität der Zellmembran aufrechterhalten (Luxy Annalen N.Y., Academy of Science 203, S. 4 (1972)). Darüberhinaus konnten membranabdichtende Eigenschaften des Vitamin E nachgewiesen werden (F. Mittelbach und G. Bodechtel, Münchener Medizinische Wochenschrift 110 (1968), 36: 1988-1993).

Es ist weiterhin bekannt, daß Vitamin E als Antioxidans insbesondere für Vitamin A geeignet ist. Vitamin A seinerseits ist ein gutes Schutzmittel, um die Schleimhaut vor Verhornung zu schützen. Darüberhinaus kann es als Infektionsschutz dienen. Vitamin-A-Mangel verursacht unter anderem Nachtblindheit (Hemeralopie) sowie gesteigerte Blendempfindlichkeit des Auges. Infolgedessen wurden Vitamin-A-haltige Mittel zur Behandlung der Augen entwickelt. Der Nachteil dieser Medikamente ist, daß Vitamin A schnell oxidiert wird und dadurch seine Wirkung herabgesetzt wird.

Aus der neueren Literatur geht hervor, daß die beiden Vitamine A und E in Wechselwirkung zueinander treten. In B. Helwig, Moderne Arzneimittel, 5. Auflage S. 1446 bis 1447 (1980) ist ausgeführt, daß der Wirkungsmechanismus des Vitamin E nur zum Teil geklärt ist. Insbesondere greift danach Vitamin E in den Umsatz und die Biosynthese von Kohlenhydraten, Eiweißkörpern, Kreatin und Nukleinsäuren ein. Die Beschleunigung der Gewebereinigung bzw. -entgiftung durch hohe Vitamin-E-Dosen wurde ebenfalls beobachtet. Bestimmte Vitamin-E-Konzentrationen und Kombinationen von Vitamin E mit anderen Wirkstoffen wurden jedoch nicht untersucht.

Neben dem Schutz vor Oxidation des Vitamin A weisen Kombinationen der Vitamine A und E die weiteren Vorteile auf, daß Vitamin A in Gegenwart von Vitamin E besser gespeichert wird und der Vitamin-A-Serumspiegel in Gegenwart von Vitamin E normalisiert wird. Tiere mit Vitamin-E-Mangel haben einen niedrigen Serumspiegel an Vitamin A, der auch bei Zugabe von hohen Vitamin-A-Dosen niedrig bleibt. Nach der Zugabe von Vitamin E normalisiert sich der Serumspiegel an Vitamin A jedoch wieder (vgl. hierzu Vitamin E as a useful therapeutic agent, Ayres S. Jr., Mihan R., J. Am. Acad. Dermatol. 7, (4) 521-5, (1982); Ayres S. Jr., Phrynoderma: Suggested Vitamin A and E Therapy (letter), Int. J. Dermatol. 22, (9) 548-9, (1983); Ayres S. Jr., Mihan R., Synergism of Vitamin A and E in achne vulgaris (letter), Int. J. Dermatol. 20, (9) 616, (1981); Ayres S. Jr., Dariers Disease: Update on an effective new therapy (letter), Arch. Dermatol., 119 (9) 710 (1983); Ayres S. Jr., Pittyriasis rubra pilaris controlled by synergism of Vitamin A and E (letter), J. Am. Acad. Dermatol. 5, (3) 350-1 (1981); Ames Sr., Factors affecting absorption, transport and storage of Vitamin A Am. J. Clin. Nutr. 22, 934 (1969).

Aufgabe der vorliegenden Erfindung ist es nunmehr, eine Verwendung zur Herstellung eines Arzneimittels zur äußerlichen Behandlung der Schleimhäute der Augen sowie der Nase und des Hals- und Rachenraumes bei Allergien und Erkältungskrankheiten bereitzustellen.

Diese Aufgabe wird dadurch gelöst, daß Vitamin E in Kombination mit einem oder mehreren durchblutungsfördernden Mitteln und/oder Vitamin A, gegebenenfalls mit einem oder mehreren Stoffen aus der Gruppe der Vitamine der B-Reihe, Vitamin C, Konservierungsstoffen, üblichen Träger- und Hilfsstoffen, zur Herstellung eines Arzneimittels zur äußerlichen Behandlung der Schleimhäute der Augen, der Nase und des Rachenraumes bei Allergien und Erkältungskrankheiten verwendet wird.

Vitamin E wirkt in dieser neuen Kombination transdermal und dringt durch das Auge in das Augeninnere ein. Daher ist das Mittel geeignet zur Behandlung der inneren Teile des Auges. Hierzu zählen insbesondere die Linse, Netzhaut, Glaskörper, Cornea und Bindehaut. Die erfindungsgemäßen Kombinationen können ebenfalls zur Behandlung der Nasenschleimhaut, des Hals- und Rachenraumes verwendet werden. Hierbei weisen die neuen erfindungsgemäß verwendeten Mittel synergistische Eigenschaften auf.

Die erfindungsgemäß verwendeten Mittel können in Form von Creme, Gel, Salbe oder Tropfen verabreicht werden. Zur Behandlung der Nasenschleimhaut können die erfindungsgemäßen Kombinationen auch in Form von Spray verwendet werden. Diese dienen insbesondere der Behandlung von Heuschnupfen und anderen Allergien, Erkältungskrankheiten sowie Schnupfen. Hierbei wird eine Kombination aus Metazolinhydrochlorid, z.B. Oxymethazolinhydrochlorid und Xylometazolinhydrochlorid mit Vitamin E, bevorzugt. Durch den Zusatz von Vitamin E werden Nebenwirkungen dieser Stoffe vermindert. Geeignet sind diese erfindungsgemäßen Kombinationen auch zur Behandlung von Entzündungen des Mundes, des Halses, des Mittelohrs und des Rachenraumes sowie Entzündungen der Stimmbänder. Als Lösungsmittel sind Alkohole, z.B. Ethanol-, Isopropanol und Propandiol sowie deren Gemische geeignet. Äther, z.B. Dimethylisosorbit, Hydroxypropylmethylcellulose oder deren Gemische kommen ebenfalls in Betracht. Ferner können Ester aliphatischer Säuren verwendet werden.

Prinzipiell können zur Herstellung von Salben die handelsüblichen Salbengrundlagen verwendet werden. Die erfindungsgemäß verwendeten Salben enthalten vorzugsweise als Grundlage:

70 bis 30 Gew.-%, vorzugsweise 60 bis 40 Gew.-% Wasser,

30 bis 5 Gew.-%, vorzugsweise 25 bis 7 Gew.-% Cetiol(R),

30 bis 2 Gew.-%, vorzugsweise 25 bis 2 Gew.-% Cetyl-Stearylalkohol oder andere aliphatische Alkohole.

Man kann anstelle von Cetyl-Stearylalkohol ganz oder teilweise auch andere emulgierende Alkohole verwenden, z.B. aliphatische Alkohole, Wollwachsalkohol, Diole, Stearinol oder Monoglyceride mit aliphatischen Säuren verestert. Um die Salbe streichfähig zu machen, kann man Paraffin oder Vaseline zusetzen. Auch Cetiol(R) kann durch andere Emulgatoren ganz oder teilweise ersetzt werden. Hier kommen beispielsweise Tween 20(R) oder Tween 80(R) in Frage. Eine besonders bevorzugte Kombination als Grundlage für die Vitamin-E-haltige Salben oder Cremes besteht aus:

30 bis 20 Gew.-% Cetyl-Stearylalkohol,

20 bis 10 Gew.-% Cetiol(R),

60 bis 40 Gew.-% Wasser.

In die erfindungsgemäß verwendeten Salben wird ein Wasser/Flüssigkeits-Emulgatoreingearbeitet. Infolge des hydrophilen Charakters kommt es zu einer gleichmäßigen Verteilung und Haftung auf der Hornhaut des Auges (Cornea). Die Wasser/Flüssigkeits-Emulsionen werden bezüglich der Haftfestigkeit noch von den Flüssig/Wasser-Emulsionen übertroffen. Als Flüssigkeits/Wasser-Emulgatoren können unter anderem Lanette N(R) und Cetiol(R) verwendet werden. Flüssigkeits/Wasser-Creme wird für Augensalben mit besonderer Tiefenwirkung benötigt; denn sie ermöglichen das Eindringen von Vitamin E sowie der eventuell weiteren zugesetzten Arzneimittel durch die Hornhaut des Auges hindurch in das Augeninnere. Eine weiter verbesserte Arzneimittelabgabe mit guter Tiefenwirkung läßt sich bei zusätzlicher Verwendung üblicher Schleimhautsalben und Polyethylenglycolsalben erreichen.

Für die Herstellung der Salben ist es von Vorteil, aseptisch zu arbeiten, d.h. die Salbengrundlage, Arzneimittel, Arbeitsgeräte und andere verwendete Gefäße müssen sterilisiert werden. Darüberhinaus ist es ratsam, den erfindungsgemäßen Mitteln Konservierungsstoffe zuzusetzen. Hierfür kommen die üblicherweise für Augensalben und Augentropfen verwendeten Stoffe in Frage. Für die Konservierung hydrophiler Augensalben und hydrophiler Cremes können unter anderem die folgenden Stoffe verwendet werden:

Benzalkoniumchlorid 0,01 Gew.-%,

Trichlorisobutanol 0,5 Gew.-%,

Phenylquecksilbernitrat 0,002 bis 0,01 Gew.-%,

Phenylquecksilberborat 0,002 bis 0,01 Gew.-%,

Natrium-Ethylquecksilberthiosalicylat 0,01 bis 0,02 Gew.-%.

Für die Konservierung lipophiler Salben und lipophiler Cremes werden folgende Stoffe bevorzugt:

Trichlorisobutanol 0,5 Gew.-%,

Benzylalkohol 0,5 Gew.-% oder

Phenylethylalkohol 4 bis 5 Gew.-%.

Das Vitamin E kann in flüssiger oder fester Form, vorzugsweise in flüssiger Form vorliegen. Als Vitamin E wird vorzugsweise freies alpha-Tocopherol, z.B. D,L-alpha-Tocopherol oder D-alpha-Tocopherol, D-alpha-Tocopherol-Konzentrat, verwendet, da die antioxidative Wirkung dieser Vitamin-E-Formen wesentlich höher ist als die anderer Vitamin-E-Derivate oder Ester.

Als Vitamin A können Vitamin-A-Palmitat, Vitamin-A-Acetat, weitere Ester des Vitamin A sowie andere Vitamin-A-Derivate eingesetzt werden.

Als weitere Zusatzstoffe kommen Ascorbinsäure sowie die Vitamine der B-Reihe, z.B. Thiaminhydrochlorid, Riboflavin-5-Phosphorsäureester, Cyanocobalamin, in Betracht. Daneben können Arzneimittel, wie Berberin-Hydrochlorid, Naphazolin und dessen Salze zugesetzt werden. Weitere nützliche Zusatzstoffe sind Dexpanthenol, Calciumpantothenat, Natrium-D-Pantothenat, Digitalis, Aesculin, Natriumiodid, Rubidiumiodid, Calciumiodid sowie verschiedene Naturstoffe. Zu letzteren zählen Fenchelöl, Rosenöl und Melissenöl. Durch diese Stoffe lassen sich der Heilungsprozeß beschleunigen und die Eigenschaften der erfindungsgemäß verwendeten Kombination verbessern. Weiterhin können Durchblutungsmittel zugesetzt werden. Hier kommen vor allem Heparin Natrium und dessen Derivate sowie hautreizende Mittel in Frage, wie Menthol, Pfefferminzöl, Oleum eukalypti, Oleum calendulae, Latschenkiefernöl, Extr. Hypocastani, Tinct. camphorae, Arnicaextrakt, Oleum camomille und Alkohole.

Um die öllöslichen Vitamine E und A in 0,9% Natriumchloridlösung zu lösen, ist es von Vorteil, einen Lösungsvermittler zu verwenden. Bei erhöhtem Gehalt an Vitamin E und A muß auch entsprechend der Lösungsvermittlergehalt erhöht werden, damit die Stabilität der Lösung gewährleistet ist. Als Lösungsvermittler sind z.B. Hydroxypropylmethylcellulose und Methocel(R) geeignet. Für die erfindungsgemäßen Tropfen können auch pflanzliche Öle Olium Olivarum, Öl Ricini, Lebertran sowie deren Derivate oder Gemische derselben verwendet werden. Für die Tropfen kommen ebenfalls die handelsüblichen Konservierungsstoffe in Frage. Für die Herstellung von Tropfen muß ebenfalls aseptisch gearbeitet werden.

In den nachfolgenden Beispielen werden die erfindungsgemäß verwendeten Mittel zur Behandlung und zum Schutz der Schleimhäute der Augen sowie der Nase, des Hals- und Rachenraumes näher erläutert:

**Beispiel 1**

Salbe aus:

EP 0 163 924 B1

| D,L-alpha-Tocopherol | 0,50 g |
|---|---|
| Retinol Palmitat | 5.000 I.E. |
| Lanette N[(R)] | 4,00 g |
| Paraffinsubliquidum | 3,00 g |
| Cetiol[(R)] | 2,00 g |
| Aqua dest. | 20,00 g |

**Beispiel 2**

Salbe aus:

| Retinol Palmitat | 100.000 I.E. |
|---|---|
| D-alpha-Tocopherol-Konzentrat | 250 mg |
| Thiaminchloridhydrochlorid | 5 g |
| Calcium pantothenat | 75 mg |
| Cetiol[(R)] | 2 g |
| Cetyl-Stearylalkohol | 2 g |
| Aqua conservata | 10,0 g |

**Beispiel 3**

Salbe aus:

| Ascorbinsäure | 50 mg |
|---|---|
| D,L-alpha-Tocopherol | 50 mg |
| Retinolacetat | 2.500 I.E. |
| Cetyl-Stearylalkohol | 3 g |
| Cetiol[(R)] | 1 g |
| Aqua conservata | 10,0 g |

**Beispiel 4**

Salbe aus:

| Retinolacetat | 100.000 I.E. |
|---|---|
| D,L-alpha-Tocopherol | 100 mg |
| Paraffinsubliquidum | 3 g |
| Alkoholum Lanae | 2 g |
| Vaselinum Album | 10,0 g |

**Beispiel 5**

Salbe aus:

5

| D,L-alpha-Tocopherol | 10 mg |
| Ergocalciferol-Retinol-Palmitat | 500 I.E. |
| Ungt. Alkoholum Lanae | 10,0 g |

**Beispiel 6**

Salbe aus:

| Dexpanthenol | 500 mg |
| D-alpha-Tocopherol-Konzentrat | 200 mg |
| Retinol Palmitat | 5.000 I.E. |
| Paraffinsubliquidum | 2 g |
| Alkoholum Lanae | 3 g |
| Vaselinum Album | 10,0 g |

**Beispiel 7**

Tropfen aus:

```
Retinol Palmitat                    10.000 I.E.

D-alpha-Tocopherol-Konzentrat       50 mg

Hydroxypropylmethylcellulose        3 g

Konservierungsmittel:

Benzalkoniumchlorid                 0,1 mg

0,9% NaCl-Lösung                    10,0 g
```

**Beispiel 8**

Tropfen aus:

| Retinol Palmitat | 100.000 I.E. |
| D,L-alpha-Tocopherol | 150 mg |
| Cetalkoniumchlorid | 0,1 mg |
| Pflanzliches öl | 10,0 g |

**Beispiel 9**

Tropfen aus:

| Retinol Palmitat | 10.000 I.E. |
|---|---|
| Berberin-HCl | 2,5 mg |
| Tetryzolin-HCl | 2,5 g |
| Hydroxypropylmethylcellulose | 3 g |
| D-alpha-Tocopherol-Konzentrat | 35 mg |
| 0,9% NaCl-Lösung | 10,0 g |

**Beispiel 10**

Tropfen aus:

| Retinolacetat | 10.000 I.E. |
|---|---|
| Actinoquinolnatrium | 50 mg |
| D,L-alpha-Tocopherol | 25 mg |
| Naphazolinnitrat | 0,75 mg |
| Hydroxypropylmethylcellulose | 3 g |
| 0,9% NaCl-Lösung | 10,0 g |

**Beispiel 11**

Salbe aus:

| D,L-alpha-Tocopherol-Konzentrat | 0,35 g |
|---|---|
| Lanette N[R] | 3,00 g |
| Paraffinsubliquidum | 3,00 g |
| Cetiol[R] | 1,00 g |
| Aqua dest. | 20,00 g |
| Heparin Natrium | 10.000 I.E. |

**Beispiel 12**

Beispiel gemäß Beispiel 1
jedoch mit 13.000 I.E. Heparin Natrium

**Beispiel 13**

Beispiel gemäß Beispiel 2
jedoch mit 6.000 I.E. Heparin Natrium

**Beispiel 14**

Beispiel gemäß Beispiele 11, 12 und 13
jedoch mit D-alpha-Tocopherolacetat
anstelle von D-alpha-Tocopherol-Konzentrat

**Beispiel 15**

Beispiel gemäß Beispiel 7
jedoch mit 6.000 I.E. Heparin Natrium

7

**Beispiel 16**

| D-alpha-Tocopherol-Konzentrat | 35 mg |
| Retinol Palmitat | 5.000 I.E. |
| Heparin Natrium | 5.000 I.E. |
| Hydroxymethylcellulose | 3,0 g |
| 0,9% Natriumchlorid | 10,0 g |

**Beispiel 17**

| D-alpha-Tocopherol-Konzentrat | 100 mg |
| Hydroxypropylmethylcellulose (Methocel$^{(R)}$) | 4 g |
| Phenylmercuriborat | 0,2 mg |
| 0,9% NaCl-Lösung | 10,0 g |
| Heparin Natrium | 6.500 I.E. |

**Beispiel 18**

Beispiel gemäß Beispiel 17
jedoch D-alpha-Tocopherolacetat
anstelle des Konzentrats zu verwenden

**Beispiel 19**

Beispiel gemäß Beispiel 18
jedoch D,L-alpha-Tocopherolacetat
anstelle von D-alpha-Tocopherolacetat zu verwenden.

**Patentansprüche**

1. Verwendung von Vitamin E in Kombination mit einem oder mehreren durchblutungsfördernden Mitteln und/oder Vitamin A, gegebenenfalls einem oder mehreren Stoffen aus der Gruppe der Vitamine der B-Reihe, Vitamin C, Konservierungsstoffe, üblichen Träger- und Hilfsstoffen zur Herstellung eines Arzneimittels zur äußerlichen Behandlung der Schleimhäute der Augen, der Nase und des Hals- und Rachenraums bei Allergien und Erkältungskrankheiten.

2. Verwendung nach Anspruch 1 mit Vitamin E in flüssiger Form.

3. Verwendung nach einem der Ansprüche 1 oder 2 mit Vitamin E in Form des freien alpha-Tocopherols.

4. Verwendung nach einem oder mehreren der Ansprüche 1 bis 3 mit Vitamin E in Kombination mit Heparin-Natrium als durchblutungsförderndes Mittel.

5. Verwendung nach einem oder mehreren der Ansprüche 1 bis 4, wobei zusätzlich ein oder mehrere Zusätze aus der Gruppe Berberin-Hydrochlorid, Naphazolin oder dessen Salze, Dexapanthenol, Calciumpantothenat, Na-D-Pantothenat, Digitalis, Aesculin, Natriumiodid, Rubidiumiodid, Fenchelöl, Rosenöl und Melissenöl eingesetzt werden.

6. Verwendung nach einem oder mehreren der Ansprüche 1 bis 5, wobei das Arzneimittel in Form von Creme, Gel, Salbe, Tropfen, Lösung oder Spray vorliegt.

7. Verwendung nach einem oder mehreren der Ansprüche 1 bis 6, wobei für Salben eine Grundlage eingesetzt wird, die aus 70 bis 30 Gew.-% Wasser, 30 bis 5 Gew.-% Cetiol (Oleyloleat) und 30 bis 2 Gew.-% Cetylstearylalkohol besteht.

8. Verwendung nach einem oder mehreren der Ansprüche 1 bis 7, wobei für hydrophile Cremes oder Salben zur Konservierung zusätzlich 0,01 Gew.-% Benzalkoniumchlorid, 0,5 Gew.-% Trichlorisobutanol, 0,002 bis 0,01 Gew.-% Phenylquecksilbernitrat, 0,002 bis 0,01 Gew.-% Phenylquecksilberborat oder 0,01 bis 0,02 Gew.-% Natriumethylquecksilberthiosalicylat eingesetzt werden.

9. Verwendung nach einem oder mehreren der Ansprüche 1 bis 8, wobei für lipophile Cremes oder Salben zur Konservierung zusätzlich 0,5 Gew.-% Trichlorisobutanol, 0,5 Gew.-% Benzylalkohol oder 4 bis 5 Gew.-% Phenylethylalkohol eingesetzt werden.

## Claims

1. The use of vitamin E in combination with one or more blood circulation-promoting agent(s) and/or vitamin A, optionally with one or more substances from the group of the vitamins of the B series, vitamin C, preservatives, conventional carriers and excipients for the preparation of a medicament for the external treatment of the mucous membranes of the eyes, of the nose and of the throat and pharynx region in cases of allergies and colds.

2. The use according to claim 1 of vitamin E in the liquid state.

3. The use according to anyone of claims 1 or 2 of vitamin E in the form of free alpha-tocopherol.

4. The use according to anyone or more of claims 1 to 3 of vitamin E in combination with heparin sodium as the blood circulation-promoting agent.

5. The use according to anyone or more of claims 1 to 4, wherein one or more additives are further employed, said additives having been selected from the group of berberine hydrochloride, naphazoline or the salts thereof, dexapanthenol, calcium pantothenate, sodium D-pantothenate, digitalis, aesculine, sodium iodide, rubidium iodide, fennel oil, rose oil and melissa oil.

6. The use according to anyone or more of claims 1 to 5, wherein the medicament is in the form of a cream, a gel, an ointment, drops, a solution or a spray.

7. The use according to anyone or more of claims 1 to 6, wherein for an oinment a base is employed which consists of from 70 to 30% by weight of water, from 30 to 5% by weight of Cetiol (oleyl oleate), and from 30 to 2% by weight of cetyl-stearyl alcohol.

8. The use according to anyone or more of claims 1 to 7, wherein 0.01% by weight of benzalkonium chloride; 0.5% by weight of trichloroisobutanol; 0.002 to 0.01% by weight of phenylmercury nitrate; 0.002 to 0.01% by weight of phenylmercury borate or 0.01 to 0.02% by weight of sodium ethylmercury thiosalicylate are additionally employed for preserving a hydrophilic cream or ointment.

9. The use according to anyone or more of claims 1 to 8, wherein 0.5% by weight of trichloroisobutanol; 0.5% by weight of benzyl alcohol or 4 to 5% by weight of phenylethyl alcohol are additionally employed for preserving lipophilic ointments and creams.

## Revendications

1. Utilisation de la vitamine E, combinée à une ou plusieurs substances favorisant la circulation du sang et/ ou à de la vitamine A, éventuellement une ou plusieurs substances choisies dans le groupe comprenant les vitamines de la série B, la vitamine C, des agents de conservation, des excipients et adjuvants usuels, pour la préparation d'un médicament pour le traitement externe des muqueuses des yeux, du nez et de la région de la gorge et du pharynx lors d'allergies et de maladies dues à un refroidissement.

2. Utilisation selon la revendication 1, avec de la vitamine E sous forme liquide.

9

3. Utilisation selon la revendication 1 ou 2, avec de la vitamine E sous la forme de l'alpha-tocophérol libre.

4. Utilisation selon l'une ou plusieurs des revendications 1 à 3, avec de la vitamine E combinée à de l'héparine sodique comme substance favorisant la circulation du sang.

5. Utilisation selon l'une ou plusieurs des revendications 1 à 4, dans laquelle on utilise, en outre, un ou plusieurs additifs choisis dans le groupe comprenant le chlorhydrate de berbérine, la naphazoline ou ses sels, le dexpanthénol, le pantothénate de calcium, le D-pantothénate de sodium, la digitaline, l'esculine, l'iodure de sodium, l'iodure de rubidium, l'essence de fenouil, l'essence de rose et l'essence de mélisse.

6. Utilisation selon l'une ou plusieurs des revendications 1 à 5, dans laquelle le médicament se présente sous forme de crème, de gel, de pommade, de gouttes, de solution ou de spray.

7. Utilisation selon l'une ou plusieurs des revendications 1 à 6, dans laquelle on utilise, pour des pommades, un produit de base qui est composé de 70 à 30 % en poids d'eau, 30 à 5 % en poids de cétiol (oléate d'oléyle) et 30 à 2 % en poids d'alcool cétylstéarylique.

8. Utilisation selon l'une ou plusieurs des revendications 1 à 7, dans laquelle on utilise en outre, pour la conservation de crèmes ou pommades hydrophiles, 0,01 % en poids de chlorure de benzalkonium, 0,5 % en poids de trichloro-isobutanol, 0,002 à 0,01 % en poids de nitrate de phénylmercure, 0,002 à 0,01 % en poids de borate de phénylmercure ou 0,01 à 0,02% en poids de thiosalicylate d'éthylmercure sodique.

9. Utilisation selon l'une ou plusieurs des revendications 1 à 8, dans laquelle on utilise en outre, pour la conservation de crèmes ou pommades lipophiles, 0,5 % en poids de trichloro-isobutanol, 0,5 % en poids d'alcool benzylique ou 4 à 5 % en poids d'alcool phényléthylique.